# EUROPEAN PATENT SPECIFICATION

(11) **EP 1 896 397 B1**
(45) Date of publication and mention of the grant of the patent: **04.11.2009**
(21) Application number: 06765547.2
(22) Date of filing: 09.06.2006
(51) Int. Cl.: C07C 253/30, C07C 255/54

(54) **PROCESS FOR PRODUCING ETHERS**
VERFAHREN ZUR HERSTELLUNG VON ETHERN
PROCEDE DE PRODUCTION D'ETHERS

(30) Priority: 22.06.2005 US 692935 P
(43) Date of publication of application: 12.03.2008
(73) Proprietor: Warner-Lambert Company LLC, New York, NY 10017 (US)
(72) Inventor: KISER, Eric Jason, Pfizer Global Res. and Dev., Ann Arbor, MI 48105 (US)
(74) Representative: Pringot, Thomas
(86) International application number: PCT/IB2006/001636
(87) International publication number: WO 2006/136910

(56) References cited:
- WO-A-2005/080320
- STEEL, WILLIAM H. ET AL: "Molecular Rulers: New Families of Molecules for Measuring Interfacial Widths" JOURNAL OF THE AMERICAN CHEMICAL SOCIETY, 124(17), 4824 -4831 CODEN: JACSAT; ISSN: 0002-7863, 2002, XP002398885
- BERNASCONI, CLAUDE F. ET AL: "Intermediates in nucleophilic aromatic substitution. 17. Kinetics of spiro Meisenheimer complexes. Effect of ring size" JOURNAL OF ORGANIC CHEMISTRY , 42(21), 3387 -93 CODEN: JOCEAH; ISSN: 0022-3263, 1977, XP002398886
- SCHMITTLING, ELISABETH A. ET AL: "Synthesis of diaryl ethers, diaryl thioethers, and diarylamines mediated by potassium fluoride-alumina and 18-crown-6" JOURNAL OF ORGANIC CHEMISTRY, 58(12), 3229 -30 CODEN: JOCEAH; ISSN: 0022-3263, 1993, XP002398887

## Description

### FIELD OF THE INVENTION

The present invention is directed to a new process for producing the compound, 4-(4-hydroxy-1-methyl-pentyloxy)-2-triftuoromethyl-benzonitrile, any of its enantiomers, any of its diastereomers, or a salt of any such compound.

### BACKGROUND OF THE INVENTION

Commonly assigned, co-pending, United States-patent application serial number 11/053,010 (filed February 13, 2004), corresponding to WO 2005/080320, discloses a number of benzonitrile derivatives and their use as anti-androgens. Example 31 A exemplifies the production of one such compound, (1S,4S)-4-(4-hydroxy-1-methylpentyoxy)-2-triftuoromethyl-benzonitrile-(3), using the synthetic route described below:

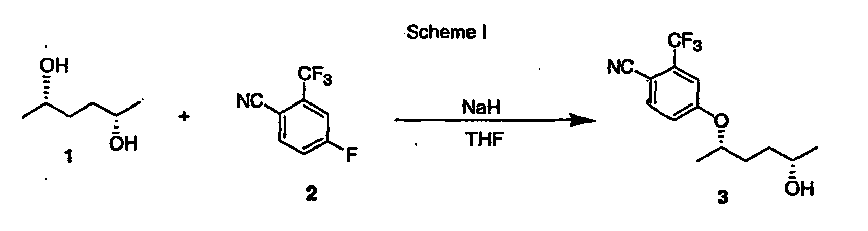

As depicted above, (2S,5S)-(+)-2,5-hexanediol (1) was dissolved in anhydrous THF and deprotonated with the strong-base, sodium hydride, under anhydrous conditions, forming an alkoxide. 4-Fluoro-2-trifluoromethylbenzonitrile (2) was then added to the reaction, maintaining anhydrous conditions, and the reactants were stirred to reaction completion. While the reaction sequence depicted above produced substantial amounts of the desired-benzonitrite of structure (3), it also produced substantial amounts of a bis-ether as described by structure (4) below. The molar ratio of compound (3) to compound (4) produced at bench scale was typically about 3:1.

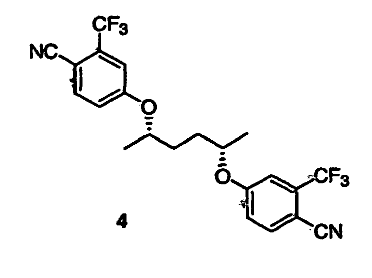

Initial attempts to scale up the reaction depicted above in Scheme I, produced mixed results. HPLC analysis showed the reaction produced a 1:1 admixture of compound (**3**) to compound (**4).** Subsequent purification produced a final yield of (3) of only 35%. Thus, a need in the art exists for processes producing higher yields of 4-(4-hydroxy-1-methyl-pentyloxy)-2-trifluoromethylbenzonitrile.

Steel et al., J. Am. Chem. Society 124, 4824-4831 (2002) and Bernasconi et al., J. Org. Chem. 42, 3387-3393 (1997) disclose processes 3387-3393 (1997) disclose processes wherein an alkanediol is monoetherified by treatment with a phenyl halide.

### SUMMARY OF THE INVENTION

A new process for producing 4-(4-hydroxy-1-methyl-pentyloxy)-2-trifluoromethyl-benzonitrile, its enantiomers, its diastereomers, and its salts has been discovered. The new process is depicted below in Reaction Scheme II:

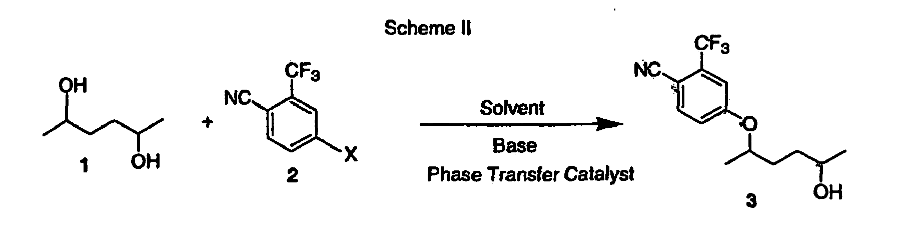

One of the reactants is the diol of structure (**1**). The other reactant is the benzonitrile of structure (**2**), in which X is represented by a halogen atom. Typically, equivalent amounts of the benzonitrile and the diol are-contacted with a base, and a phase transfer catalyst, in a solvent for a sufficient period of time to allow the reaction to proceed to completion.

The new process possesses a number of advantages. It substantially reduces generation of the undesired bis-ether of structure (**4**). Since the new process can optionally be run under hydrous conditions, it is much more amendable to industrial scale-up. The process is much safer than the hydride method since sodium hydride reacts with the diol (**1**) producing one mole of hydrogen gas for every mole of (**1**) used in the reaction. The former process also requires anhydrous conditions because sodium hydride reacts violently with water producing hydrogen gas. The flammability, high reactivity and generally dangerous nature of sodium hydride make it undesirable for large scale work. This new process is much more amenable to industrial applications.

### DETAILED DESCRIPTION OF THE INVENTION

The invention is directed to a new process for producing the compound 4-(4-hydroxy-1-methyl-pentyloxy)-2-trifluoromethyl-benzonitrile (hereinafter the "compound"), whose structure is depicted below:

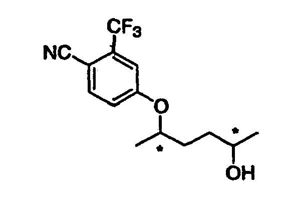

The compound contains two chiral centers, which are marked with asterisks (*). The invention should be construed as being directed to the production of the racemate, an individual enantiomer, an admixture of enantiomers, an admixture of diastereomers, or any combination thereof. The compound also contains a hydroxyl function, which may be converted to a salt, and more typically to a pharmaceutically acceptable base addition salt. Any reference in this application to the compound should be construed as referring to the racemate, an individual enantiomer, mixtures of enantiomers, mixtures of diastereomers and/or combinations thereof, or a salt of any such entity.

As used in the application, the term "salts" is intended to refer to pharmaceutically acceptable salts and to salts suitable for use in industrial processes. The term "pharmaceutical acceptable" means suitable for administration to a mammal. Examples of pharmaceutically acceptable salts include any non-toxic organic or inorganic basic addition salt of the compound. Illustrative bases which form suitable salts include alkali metal or alkaline-earth metal hydroxides such as sodium, potassium, calcium, magnesium, or barium hydroxides; ammonia, and aliphatic, alicyclic, or aromaticorganic amines such as methylamine, dimethylamine, trimethylamine, and picoline.

The process for producing 4-(4-hydroxy-1-methyl-pentyloxy)-2-trifluoromethyl-benzonitrile was described above in Reaction Scheme II and is repeated below for the reader's convenience.

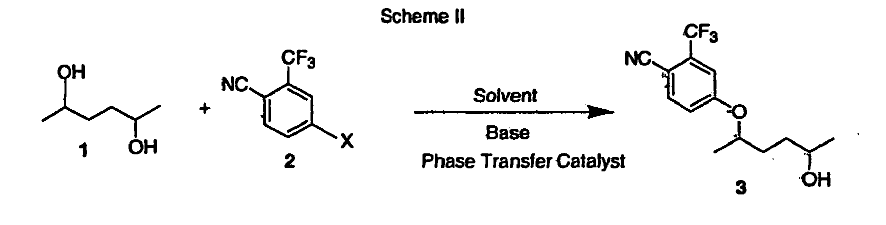

One of the reactants is the diol of formula (**1**), which is commercially available. This diol has two asymmetric centers and exists as three (3) separate stereoisomers (two enantiomers and one meso form). It is possible to control the stereochemistry of the final product by utilizing a single stereoisomer in the reaction. For example, utilizing the (R,R)-diol produces the (R,R) isomer of (**3**) and utilizing the (S,S)-diol produces the-(S,S)-isomer of (**3**). Use of the (R,S) diol (a meso form) produces a racemic mixture of the (R,S) and (S,R) isomers of (3). Typically, the alcohol stereoisomer will be (2S,5S)-(+)-2,5-hexanediol (which is commercially available) which generates (1S,4S)-4-(4-Hydroxy-1-methyl-pentyloxy)-2-trifluoromethyl-benzonitrile as the final product. Alternatively, the final product of structure (**3**) derived from any mixture of stereoisomers of diol (1) can be subjected to a chiral separation to produce the desired stereoisomer.

The other reactant is the benzonitrile of structure (2) in which X is a halogen atom. Typically X will be fluorine. These benzonitriles are available from commercial sources.

The reaction is typically carried out by contacting the diol -(1) and the benzonitrile (2) in a solvent. The quantity of these reactants is not-critcal. For example, for every mole of benzonitrile used, from about-0.1 to 100 moles of diol may be used, more typically about 0.1 to about 3 moles of diol, arid usually about 0.9 to about 1.1 moles of diol. Typically equivalent amounts will be used.

Any number of standard industrially suitable solvents maybe used in the reaction (as long as they are compatible with the base used). Examples of such solvents include organic solvents like tetrahydrofuran, diethyl ether, methyl-tert-butyl ether (and other acyclic and cyclic alkyl ethers), pentane, hexane, heptane, cyclohexane(and other acyclic and cyclic alkanes), dichloromethane, dichloroethane (and other chlorinated alkanes), toluene, benzene, xylene (and other aromatic hydrocarbons), acetone, methyl ethyl ketone (and other alkanones), ethyl acetate, propyl acetate (and other alkyl alkanoates), methanol, ethanol, propanol (and other alcohols), acetonitrile, propionitrile (and other alkyl cyanides), dimethyl sulfoxide, dimethyl formamide, dimethyl acetamide, N-methylpyrrolidone, chlorobenzene, etc. The above list of organic solvents is being presented to illustrate the invention, not to limit the scope-of the claims. The absence of a particular solvent from the list above is not an indication that its use should be excluded from the invention. Any organic solvent suitable for use in an industrial process should be considered to be encompassed the term "organic solvent" The quantity of organic solvent can vary widely as known to those skilled in the art .

The solvent may also be water. Alternatively, the solvent maybe an admixture of water and an organic solvent. The ratio of water to organic solvent can vary widely. For example it may range from 100% water to 100% organic solvent, more typically it will range from 50 v/v% to 90 v/v % organic solvent, the remainder of the solvent being water. In a further embodiment it will range from 50 v/v % to 90 v/v% water (with the rest being one, or more, organic solvents)

The reactants are also contacted with a base. The particular base is not critical as long as it is compatible with the solvent used. Examples of suitable bases include sodium hydroxide, potassium hydroxide, lithium hydroxide, barium hydroxide, cesium hydroxide (i.e. metal hydroxide bases), sodium carbonate, potassium carbonate, lithium carbonate (i.e. metal carbonate bases), potassium bicarbonate, sodium bicarbonate (i.e. metal bicarbonate bases), ammonia, triethylamine (i.e. amine bases), sodium acetate, potassium acetate (i.e. metal carboxylate bases), sodium methoxide, sodium ethoxide, sodium tert-butoxide (i.e. metal alkoxide bases). The above list of bases is being presented to illustrate the invention, not to limit the scope of the claims. The absence of a particular base from the list above is not an indication that its use should be excluded from the invention. Any base suitable for use in a chemical process should be considered to be encompassed the term "base".

The base may be present as a solid, it maybe added to the reaction as a liquid, or it may be bubbled into the reaction as a gas. The quantity of base utilized can vary widely and may range from about 0.1 equivalents to about 100 equivalents. From a process standpoint, 1.0 equivalent to 10 equivalents is usual, with 5.0 equivalents being typical.

The reaction will be carried out in the presence of a phase transfer catalyst. Phase transfer catalysis refers to the phenomenon of rate enhancement of a reaction between chemical species located in different phases (immiscible liquids or solid and liquid) by addition of a small quantity of an agent (called the "phase-transfer catalyst") that extracts one of the reactants, most commonly an anion, across the interface into the other phase so that reaction can proceed. These catalysts are salts of "onium ions"(e.g. tetraalkylammonium salts and tetraalkylphosphonium salts) or agents that complex inorganic cations (e.g. crown ethers). The catalyst cation is not consumed in the reaction although an anion exchange can occur.

As those skilled in the art recognize, a large number of phase transfer catalysts have been described in the literature. Many of these catalysts are commercially available. Any phase transfer catalyst may be utilized in the invention. The literature typically classifies these phase transfer-catalysts into one of four categories, based upon chemical structure. These categories include 1) quaternary ammonium compounds, 2) quaternary phosphonium compounds, 3) crown ethers and 4) polyethylene glycol derivatives.

Examples of suitable quaternary ammonium compounds include, but are not limited to: benzyl tributyl ammonium bromide, benzyl tributyl ammonium chloride, benzyl triethyl ammonium bromide, benzyl triethyl ammonium chloride, benzyl trimethyl ammonium chloride, cetyl pyridinium chloride, cetyl trimethyl ammonium bromide, didecyl dimethyl ammonium chloride, dimethyl distearyl ammonium bisulfate, dimethyl distearyl ammonium methosulfate, dodecyl trimethyl ammonium bromide, dodecyl trimethyl ammonium chloride, methyl tributyl ammonium chloride, methyl tributyl ammonium chloride, methyl tributyl ammonium hydrogen sulfate, methyl tricaprylyl ammonium chloride, methyl trioctyl ammonium chloride, myristyl trimethyl ammonium bromide, cetyl pyridinium bromide, phenyl trimethyl ammonium chloride, tetrabutyl ammonium borohydride, tetrabutyl ammonium bromide, tetrabutyl ammonium chloride, tetrabutyl ammonium fluoride, tetrabutyl ammonium hydrogen sulfate, tetrabutyl ammonium hydroxide, tetrabutyl ammonium iodide, tetrabutyl ammonium perchlorate, tetraethyl ammonium bromide, tetraethyl ammonium chloride, tetraethyl ammonium hydroxide, tetrahexyl ammonium bromide, tetrahexyl ammonium iodide, tetramethyl ammonium bromide, tetramethyl ammonium chloride, tetramethyl ammonium fluoride, tetramethyl ammonium hydroxide, tetramethyl ammonium iodide, tetraoctyl ammonium bromide, tetrapropyl ammonium bromide, tetrapropyl ammonium chloride, tetrapropyl ammonium hydroxide, tetraethyl ammonium chloride, tetraethyl ammonium hydroxide, tetramethyl ammonium bromide, tetramethyl ammonium chloride, tetramethyl ammonium fluoride, tetramethyl ammonium hydroxide, tetraoctyl ammonium bromide, tetrapropyl ammonium bromide, tetrapropyl ammonium chloride, tetrapropyl ammonium hydroxide, tributyl methyl ammonium chloride, triethyl benzyl ammonium chloride.

Examples of suitable quaternary phosphonium salts include, but are not limited to: benzyl triphenyl phosphonium bromide, benzyl triphenyl phosphonium chloride, butyl triphenyl phosphonium bromide, butyl triphenyl phosphonium chloride, ethyl triphenyl phosphonium acetate, ethyl triphenyl phosphonium bromide, ethyl triphenyl phosphonium iodide, methyl triphenyl phosphonium bromide, tetrabutyl phosphonium bromide, tetraphenyl phosphonium bromide.

Examples of suitable polyethylene glycols include: monoethylene glycol dimethyl ether, diethylene glycol dimethyl ether, triethylene glycol dimethyl ether, tetraethylene glycol dimethyl ether, dipropylene glycol dimethyl ether, dimethyl glycol dibutyl ether, polyethylene glycol dibutyl ether, and diethylene glycol dibutyl ether.

Examples of crown ethers include: 18-crown-6, dibenzo 18-crown-6, dicyclohexyl-18-crown-6, 12-crown-4, 13-crown-4, 15-crown-5, and 27-crown-9.

Typically, only one phase transfer catalyst will be used, but multiple phase transfer catalysts may be added, if desired. An effective amount of a phase transfer catalyst will be added to the reaction, typically at the beginning of the reaction. An effective amount can vary depending upon the specific catalyst used, but as a general guideline from about 0.01 to about 1000 mole percent of catalyst will be used, with about 1-25 mole percent being a typical range.

The order of addition of the reactants is not critical. Typically, the diol (1) and the benzonitrile (2) are contacted with the solvent. The base and the phase transfer catalyst are then added to the reaction mixture. The reaction may be carried out at a temperature ranging from about 0 °C to reflux, typically about room temperature. The reaction is carried out for a sufficient period of time to allow its completion (i.e. 1 minute to 24 hours depending upon the quantities of reactants). Typically the progress of the reaction will be monitored by high performance liquid chromatography (HPLC) or other similar methodology.

The final product of structure (3) can be isolated and recovered as is known in art. For example, the final product can be recovered by extraction, evaporation, or other techniques known in the art. It may optionally be purified by chromatography, recrystallization, distillation, or other techniques known in the art.

### EXAMPLES

The following examples are being presented in order to further illustrate the invention. The invention and the corresponding claims should not be construed as being limited to the embodiments exemplified in these experiments. All HPLC analyses described below were carried out in the following manner: Carried out on an Aglient HP 1100 Series Instrument with the following parameters:
Column: Zorbax Eclipse® XDB-C8; 4.6 mm x 150 mm; 5 micron
Mobile Phase A: Water with 0.2% perchloric acid
Mobile Phase B: Acetonitrile
Gradient: 30% B to 95% B over 10 min. Hold at 95% B for 10 min. Return to 30% B over 1 min.
Flow Rate: 1 ml/min.
Wavelength: 215 nm
Injection Volume: 5 ul
Column Temp.: 30°C
Sample Diluent: 50:50 ACN:H₂O
Retention time of (3) =7.5 min (i.e. final product)
Retention time of (4) =11.1 min (i.e. the bis-ether by- product)

### EXAMPLE 1

This example illustrates the preparation of 4-[(1S,-4S)-4-hydroxy-1-methylpentyloxy]-2-trifluoromethylbenzonitrile, using a base in a system containing an organic solvent and water. It also illustrates the use of a phase transfer catalyst.

A 3 L, 4-necked flask was equipped with a mechanical stirrer and a J-KEM temperature probe. The flask was charged with 100.0 g (0.529 mol) of 4-fluoro-2-(trifluoromethyl)benzonitrile, 68:40 g (0.579 mol) of (2S,5S)-(+)-2,5-hexanediol, 9.03 g (0.027 mol) of tetrabutylammonium bisulfate and 1.0 L of toluene. The mixture was shirred and 0.230 L of 45 wt% potassium hydroxide was added. A slight exotherm from 14°C to 20°C was observed. The 2 phase mixture was stirred overnight, at which time HPLC analysis indicated complete reaction and a 10:1 ratio of desired product to bis ether adduct (i.e. Compound 3 to Compound 4). Water (1.0 L) was added and the layers were separated. The organic layer was washed with 3 x0.25 L of water and 1 × 0.25 L of brine, dried over magnesium sulfate, filtered through Celite, and then evaporated at the Rotavap. The crude product was isolated as 158.12 g (104%) of clear, almost colorless oil. Purification was achieved by flash chromatography on silica gel using a Biotage system (hexanes/ethyl acetate). This yielded 116.75-g (77%) of 4-[(1S,4S)-4-hydroxy-1-methylpentyloxy]-2-trifluoromethylbenzonitrile as clear, colorless oil. The material assayed at >99% purity (a/a, HPLC) and had a proton NMR spectrum consistent with structure.

### EXAMPLE 2

This example illustrates the preparation of 4,[(1S,4S)-4-hydroxy-1-methylpentyloxy]-2-trifluoromethylbenzonitrile, using a base in a mixture of an organic solvent and water. A phase transfer catalyst was used.

A 50 mL reaction tube was charged with 257 mg (1.3 mmol) of 4-fluoro-2-(trifluoromethyl)benzonitrile, 183 mg(1.4 mmol) of (2S;5S)-(+)-2,5-hexanediol, 22 mg of tetrabutylamnionium bromide and 2.5 mL of toluene. Potassium hydroxide solution (45 wt%, 6.6 mmol) was added and the two-phase mixture was stirred vigorously and monitored by HPLC until the starting benzonitrile had been consumed (< 1 area%). The reaction was allowed to proceed at ambient temperature for approximately 75 minutes. HPLC analysis of organic phase showed 1 area% of unreacted benzonitrile, 89 area% of the desired final product and 8.9 area% of the bis-ether adduct. The final product was not isolated because the purpose of the experiment was to determine the ratio of product (Compound 3) to bis-ether adduct (Compound 4).

### EXAMPLE 3

A 50 mL reaction tube was charged with 262 mg (1.3 mmol) of 4-fluoro-2-(trifluoromethyl)benzonitrile, 181 mg( 1.4 mmol) of (2S;5S)-(+)-2,5-hexanediol, 25 mg of cetyltrimethylammonium bromide and 2.5 mL of toluene. Potassium hydroxide solution (45 wt%, 6.6 mmol) was added and the two-phase mixture was stirred vigorously and monitored by HPLC until the starting -benzonitrile had been consumed (< 1 area%). The reaction was allowed to proceed at ambient temperature for approximately 75 minutes. HPL-C analysis of organic phase showed 31 area % of unreacted benzonitrile, 67 area % of the desired final product and 0.85 area % of the bis-ether adduct. The reaction was allowed to proceed an additional 10 days over a vacation, HPLC analysis of the organic phase showed 88 area % of the desired final product and 9.2 area % of the bis-ether adduct. The final product was not isolated because the purpose of the experiment was to determine the ratio of product (Compound 3) to bis ether adduct (Compound **4**).

### EXAMPLE 4

A 25ml reaction tube was charged with 1022 mg -(5.4 mmol) of 4-fluoro-2-(trifluoromethyl)benzonitrile, 699 mg (5.9 mmol) of (2S,5S)-(+)-2,5-hexanediol, 96 mg (0.28 mmol) of tetrabutylammonium bisulfate and 10 mL of toluene. Stirred vigorously and then added 1.40 mL of 50 wt% NaOH. The reaction was stirred at ambient temperature for 75 minutes. HPLC analysis of the organic phase showed a 7.0:1 mix (mole:mole) of final product to bis-ether adduct. The final product was not recovered because the purpose the experiment was to determine the ratio of product (Compound 3) to bis-ether adduct (Compound **4**).

### COMPARATIVE EXAMPLE A

This example illustrates the preparation of 4-[(1S,4S)-4-Hydroxy-1-methylpentyloxy]-2-trifluoromethylbenzonitrile, using a strong base under anhydrous conditions, as described in co-pending United States patent application serial number 11/053,010.

A 10 L, jacketed ChemGlass reactor was fitted with a mechanical stirrer, nitrogen sweep line, and NesLab chiller. The vessel was purged with nitrogen for 15 minutes and then charged with 61 g (1.5 mol) of sodium hydride (60% in mineral oil) and 2.0 L of tetrahydrofuran ("THF"). The suspension was stirred and cooled to 6.5°C and then a solution of 180.3 g (1.53 mol) of (2S,5S)-(+)-2,5-hexanediol in 1.0 L of THF was added, in 4 portions, over 35 minutes. The mixture became very thick and 1.0 L of THF was added about halfway through the addition in order to facilitate better stirring. The maximum internal temperature observed was 14.4°C. The suspension was stirred for 30 minutes and then 288.1 g (1:52 mol) of 4-fluoro-2-trifluoromethyl)benzonitrile was added. The mixture was further diluted with another 1.0 L of THF and then warmed to room temperature and allowed to stir overnight (a clear solution was obtained after 30 minutes). HPLC analysis at the 21 hour point showed a 48:45 mixture of the expected product (3) to the bis-alkylated by product (4). Water (3.0 L) and ethyl acetate (4.0 L) were added and the layers separated. The organic layer was washed with 2 x 2.0 L of water and 1 x 2.0 L of brine. The aqueous layers were back-extracted with 2.0 L of ethyl acetate and then the organic layers were combined and evaporated at the Rotavap. The residue was twice dissolved in 1.0 L of 2-propanol and evaporated at the Rotavap (to remove water). The-crude product was twice purified by flash chromatography on silica gel using dichloromethane/methanol. A third chromatography column (Biotage system) was carried out using ethyl acetate/hexanes. The purified target was isolated as 149.8 g (34%) of an oil. The material assayed at >99% purity (a/a, HPLC) and had a proton NMR spectrum consistent with structure.

### COMPARATIVE EXAMPLE B

This example also illustrates the preparation of 4-[(1S,4S)-4-Hydroxy-1-methylpentyloxy]-2-trifluoromethylbenzonitrile, using a strong base in THF, as described in co-pending United States patent application United States patent application serial number 11/053,010 on a smaller scale than described in Example A.

A 500 mL 3-necked flask was fitted with a mechanical stirrer, argon line and temperature probe. The flask was purged with argon and then charged with 4.237 g (177 mmol) of sodium hydride (60% in mineral oil) and 70 mL of tetrahydrofuran (THF). The grey suspension was stirred and to it was added a solution of 12.6 g (107 mmol) of (2S,5S)-2,5-hexanediol in 130 mL of THF over 20 minutes. The thick mixture was stirred for 45 minutes and then a solution of 20.0 g (106 mmol) of 4-fluoro-2-trifluoromethylbenzonitrile in 130 mL of THF was added over 5 minutes. The mixture was stirred for 2.5 hours and then refluxed for 2 hours. The reaction solution was then allowed to cool to room temperature and stir over a weekend. HPLC analysis at this point showed a 69:25 mixture of the intended product (3) to bis-alkylated byproduct (**4**). The reaction mixture was partitioned between ethyl acetate and water. The layers were separated and the organic layer was washed with water and brine and then dried over magnesium sulfate. The solvent was then removed at the Rotavap giving 35.93 g of clear oil. The product was purified by chromatography on silica gel using dichloromethane/methanol as the eluent. The product-containing fractions were pooled and evaporated at the Rotavap giving 19.63 g (65%) of (3) as a clear, colorless oil. The material assayed at 99.7% (a/a) purity by HPLC and had a proton NMR spectrum consistent with structure.

## Claims

1. A process for producing a compound of the formula: 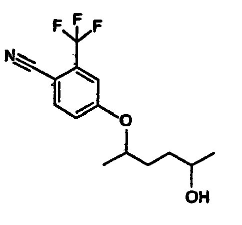 or a salt thereof, comprising reacting benzonitrile derivative of formula (i), in which X is a halogen atom, 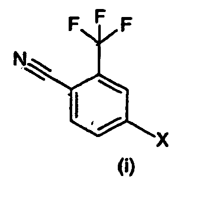 with a diol of formula (ii) 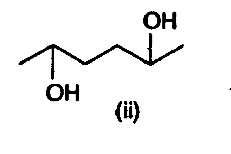 in the presence of a base and a phase transfer catalyst, at a suitable temperature, in a suitable solvent, for a sufficient period of time.

2. The process according to claim 1 in which X is fluorine.

3. The process according to claims 1 or 2 in which said base is selected from the group consisting of metal hydroxide, metal carbonate, metal bicarbonate, amine, and metal alkoxide.

4. The process according to any of claims 1, 2, or 3 in which said base is selected from the group consisting sodium hydroxide, potassium hydroxide, lithium hydroxide, barium hydroxide, cesium hydroxide, sodium carbonate, potassium carbonate, lithium carbonate, potassium bicarbonate, sodium bicarbonate, ammonia, triethylamine, sodium acetate, potassium acetate, sodium methoxide, sodium ethoxide, and sodium tert-butoxide.

5. The process according to any of claims 1, 2, 3 or 4 in which said phase transfer catalyst is selected from the group consisting of quaternary ammonium, quaternary phosphonium, crown ether, and polyethylene glycol.

6. The process according to any of claims 1, 2, 3, 4, or 5 in which said phase transfer catalyst is present in the quantity of about 0.01 mole percent to about 1000 mole percent.

7. The process according to any one of claims 5 or 6 in which said quaternary ammonium catalyst is selected from the group consisting of: benzyl tributyl ammonium bromide, benzyl tributyl ammonium chloride, benzyl triethyl ammonium bromide, benzyl triethyl ammonium chloride, benzyl trimethyl ammonium chloride, cetyl pyridinium chloride, cetyl trimethyl ammonium bromide, didecyl dimethyl ammonium chloride, dimethyl distearyl ammonium bisulfate, dimethyl distearyl ammonium methosulfate, dodecyl trimethyl ammonium bromide, dodecyl trimethyl, ammonium chloride, methyl tributyl ammonium chloride, methyl tributyl ammonium chloride, methyl tributyl ammonium hydrogen sulfate, methyl tricaprylyl ammonium chloride, methyl trioctyl ammonium chloride, myristyl trimethyl ammonium bromide, cetyl pyridinium bromide, phenyl trimethyl ammonium chloride, tetrabutyl ammonium borohydride, tetrabutyl ammonium bromide, tetrabutyl ammonium choride, tetrabutyl ammonium fluoride, tetrabutyl ammonium hydrogen sulfate, tetrabutyl ammonium hydroxide, tetrabutyl ammonium iodide, tetrabutyl ammonium perchlorate, tetraethyl ammonium bromide, tetraethyl ammonium chloride, tetraethyl ammonium hydroxide, tetrahexyl ammonium bromide, tetrahexyl ammonium iodide, tetramethyl ammonium bromide, tetramethyl ammonium chloride, tetramethyl ammonium fluoride, tetramethyl ammonium hydroxide, tetramethyl ammonium iodide, tetraoctyl ammonium bromide, tetrapropyl ammonium bromide, tetrapropyl ammonium chloride, tetrapropyl ammonium hydroxide, tetraethyl ammonium chloride, tetraethyl ammonium hydroxide, tetramethyl ammonium bromide, tetramethyl ammonium chloride, tetramethyl ammonium fluoride, tetramethyl ammonium hydroxide, tetraoctyl ammonium bromide, tetrapropyl ammonium bromide, tetrapropyl ammonium chloride, tetrapropyl ammonium hydroxide, tributyl methyl ammonium chloride, and triethyl benzyl ammonium chloride.

8. The process according to any one of claims 5 or 6 in which said quaternary phosphonium catalyst is selected from the group consisting of: benzyl triphenyl phosphonium bromide, benzyl triphenyl phosphonium chloride, butyl triphenyl phosphonium bromide, butyl triphenyl phosphonium chloride, ethyl triphenyl phosphonium acetate, ethyl triphenyl phosphonium bromide, ethyl triphenyl phosphonium iodide, methyl triphenyl phosphonium bromide, propyl triphenyl phosphonium bromide, propyl triphenyl phosphonium chloride, tetrabutyl phosphonium bromide, and tetraphenyl phosphonium bromide.

9. The process according to any one of claims 5 or 6 in which said crown ether is selected from the group consisting 18-crown-6, dibenzo 18-crown-6, dicyclohexyl-18-crown-6, 12-crown-4, 13-crown-4, 15-crown-5, and 27-crown-9.

10. The process according to any one of claims 5 or 6 in which said polyethylene glycol catalyst is selected from the group consisting of monoethylene glycol dimethyl ether, diethylene glylol dimethyl ether, triethylene glycol dimethyl ether, tetraethylene glycol dimethyl ether, dipropylene glycol dimethyl ether, diethyl glycol dibutyl ether, polyethylene glycol dibutyl ether, and diethylene glycol dibutyl ether

11. The process according to any of claims 1-10 in which said solvent is selected from the group consisting of organic solvent and water.

12. The process according to any one of claims 1-11 in which said diol has the following stereochemistry:

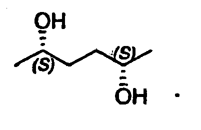

13. The process according to any one of claims 1-12 in which said compound is (1S,45)-4-(4-Hydroxy-1-methyl-pentyloxy)-2-trifluoromethyl-benzonitrile, or a pharmaceutically acceptable salt thereof.

## Patentansprüche

1. Verfahren zur Herstellung einer Verbindung der Formel: 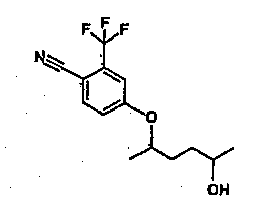 oder eines Salzes davon, umfassend Umsetzen eines Benzonitrilderivats der Formel (i), in der X ein Halogenatom ist, 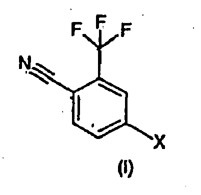 mit einem Diol der Formel (ii) 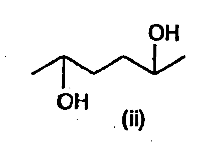 in Gegenwart einer Base und eines Phasentransferkatalysators bei einer geeigneten Temperatur in einem geeigneten Lösungsmittel für einen ausreichenden Zeitraum.

2. Verfahren gemäß Anspruch 1, wobei X Fluor ist.

3. Verfahren gemäß Anspruch 1 oder 2, wobei die Base ausgewählt wird aus der Gruppe, bestehend aus Metallhydroxid, Metallcarbonat, Metallbicarbonat, Amin und Metallalkoxid.

4. Verfahren gemäß einem der Ansprüche 1, 2 oder 3, wobei die Base ausgewählt wird aus der Gruppe, bestehend aus Natriumhydroxid, Kaliumhydroxid, Lithiumhydroxid, Bariumhydroxid, Cäsiumhydroxid, Natriumcarbonat, Kaliumcarbonat, Lithiumcarbonat, Kaliumbicarbonat, Natriumbicarbonat, Ammoniak, Triethylamin, Natriumacetat, Kaliumacetat, Natriummethoxid, Natriumethoxid und Natrium-tert.-butoxid.

5. Verfahren gemäß einem der Ansprüche 1, 2, 3 oder 4, wobei der Phasentransferkatalysator ausgewählt wird aus der Gruppe, bestehend aus quaternärem Ammonium, quaternärem Phosphonium, Kronenether und Polyethylenglycol.

6. Verfahren gemäß einem der Ansprüche 1, 2, 3, 4 oder 5, wobei der Phasentransferkatalysator in der Menge von etwa 0,01 mol-% bis etwa 1.000 mol-% vorhanden ist.

7. Verfahren gemäß einem der Ansprüche 5 oder 6, wobei der quaternäre Ammoniumkatalysator ausgewählt wird aus der Gruppe, bestehend aus: Benzyltributylammoniumbromid, Benzyltributylammoniumchlorid, Benzyltriethylammoniumbromid, Benzyltriethylammoniumchlorid, Benzyltrimethylammoniumchlorid, Cetylpyridiniumchlorid, Cetyltrimethylammoniumbromid, Didecyldimethylammoniumchlorid, Dimethyldistearylammoniumbisulfat, Dimethyldistearylammoniummethosulfat, Dodecyltrimethylammoniumbromid, Dodecyltrimethylammoniumchlorid, Methyltributylammoniumchlorid, Methyltributylammoniumchlorid, Methyltributylammoniumhydrogensulfat, Methyltricaprylylammoniumchlorid, Methyltrioctylammoniumchlorid, Myristyltrimethylammoniumbromid, Cetylpyridiniumbromid, Phenyltrimethylammoniumchlorid, Tetrabutylammoniumborhydrid, Tetrabutylammoniumbromid, Tetrabutylammoniumchlorid, Tetrabutylammoniumfluorid, Tetrabutylammoniumhydrogensulfat, Tetrabutylammoniumhydroxid, Tetrabutylammoniumiodid, Tetrabutylammoniumperchlorat, Tetraethylammoniumbromid, Tetraethylammoniumchlorid, Tetraethylammoniumhydroxid, Tetrahexylammoniumbromid, Tetrahexylammoniumiodid, Tetramethylammoniumbromid, Tetramethylammoniumchlorid, Tetramethylammoniumfluorid, Tetramethylammoniumhydroxid, Tetramethylammoniumiodid, Tetraoctylammoniumbromid, Tetrapropylammoniumbromid, Tetrapropylammoniumchlorid, Tetrapropylammoniumhydroxid, Tetraethylammoniumchlorid, Tetraethylammoniumhydroxid, Tetramethylammoniumbromid, Tetramethylammoniumchlorid, Tetramethylammoniumfluorid, Tetramethylammoniumhydroxid, Tetraoctylammoniumbromid, Tetrapropylammoniumbromid, Tetrapropylammoniumchlorid, Tetrapropylammoniumhydroxid, Tributylmethylammoniumchlorid und Triethylbenzylammoniumchlorid.

8. Verfahren gemäß einem der Ansprüche 5 oder 6, wobei der quaternäre Phosphoniumkatalysator ausgewählt wird aus der Gruppe, bestehend aus: Benzyltriphenylphosphoniumbromid, Benzyltriphenylphosphoniumchlorid, Butyltriphenylphosphoniumbromid, Butyltriphenylphosphoniumchlorid, Ethyltriphenylphosphoniumacetat, Ethyltriphenylphosphoniumbromid, Ethyltriphenylphosphoniumiodid, Methyltriphenylphosphoniumbromid, Propyltriphenylphosphoniumbromid, Propyltriphenylphosphoniumchlorid, Tetrabutylphosphoniumbromid und Tetraphenylphosphoniumbromid.

9. Verfahren gemäß einem der Ansprüche 5 oder 6, wobei der Kronenether ausgewählt wird aus der Gruppe, bestehend aus 18-Krone-6, Dibenzo-18-krone-6, Dicyclohexyl-18-krone-6, 12-Krone-4, 13-Krone-4, 15-Krone-5 und 27-Krone-9.

10. Verfahren gemäß einem der Ansprüche 5 oder 6, wobei der Polyethylenglycol-Katalysator ausgewählt wird aus der Gruppe, bestehend aus Monoethylenglycoldimethylether, Diethylenglycoldimethylether, Triethylenglycoldimethylether, Tetraethylenglycoldimethylether, Dipropylenglycoldimethylether, Diethylglycoldibutylether, Polyethylenglycoldibutylether und Diethylenglycoldibutylether.

11. Verfahren gemäß einem der Ansprüche 1 bis 10, wobei das Lösungsmittel aus der Gruppe, bestehend aus organischem Lösungsmittel und Wasser, ausgewählt wird.

12. Verfahren gemäß einem der Ansprüche 1 bis 11, wobei das Diol die folgende Stereochemie hat:

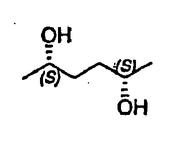

13. Verfahren gemäß einem der Ansprüche 1 bis 12, wobei die Verbindung (1S,4S)-4-(4-Hydroxy-1-methylpentyloxy)-2-trifluormethylbenzonitril oder ein pharmazeutisch verträgliches Salz davon ist.

## Revendications

1. Procédé de production d'un composé de formule : 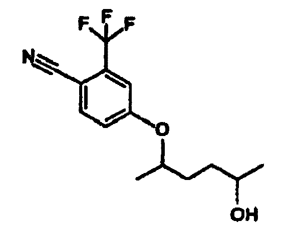 ou d'un sel correspondant, comprenant la réaction d'un dérivé benzonitrile de formule (i), dans lequel X est un atome d'halogène, 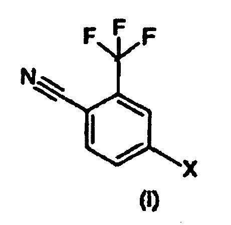 avec un diol de formule (ii) 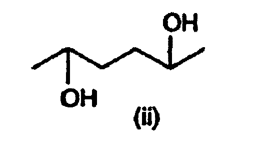 en présence d'une base et d'un catalyseur de transfert de phase, à une température convenable, dans un solvant convenable, pendant une période de temps suffisante.

2. Procédé selon la revendication 1, dans lequel X est du fluor.

3. Procédé selon la revendication 1 ou 2, dans lequel ladite base est sélectionnée dans le groupe consistant en un hydroxyde métallique, un carbonate métallique, un bicarbonate métallique, une amine et un alcoxyde métallique.

4. Procédé selon l'une quelconque des revendications 1, 2 et 3, dans lequel ladite base est sélectionnée dans le groupe consistant en l'hydroxyde de sodium, l'hydroxyde de potassium, l'hydroxyde de lithium, l'hydroxyde de baryum, l'hydroxyde de césium, le carbonate de sodium, le carbonate de potassium, le carbonate de lithium, le bicarbonate de potassium, le bicarbonate de sodium, l'ammoniaque, la triéthylamine, l'acétate de sodium, l'acétate de potassium, le méthanolate de sodium, l'éthanolate de sodium et le *tert*-butanolate de sodium.

5. Procédé selon l'une quelconque des revendications 1, 2, 3 et 4, dans lequel ledit catalyseur de transfert de phase est sélectionné dans le groupe consistant en un ammonium quaternaire, un phosphonium quaternaire, un éther-couronne et un polyéthylèneglycol.

6. Procédé selon l'une quelconque des revendications 1, 2, 3, 4 et 5, dans lequel ledit catalyseur de transfert de phase est présent en une quantité allant d'environ 0,01 pour cent en moles à environ 1000 pour cent en moles.

7. Procédé selon l'une quelconque des revendications 5 et 6, dans lequel ledit catalyseur ammonium quaternaire est sélectionné dans le groupe consistant en : le bromure de benzyltributylammonium, le chlorure de benzyltributylammonium, le bromure de benzyltriéthylammonium, le chlorure de benzyltriéthylammonium, le chlorure de benzyltriméthylammonium, le chlorure de cétylpyridinium, le bromure de cétyltriméthylammonium, le chlorure de didécyldiméthylammonium, le bisulfate de diméthyldistéarylammonium, le méthosulfate de diméthyldistéarylammonium, le bromure de dodécyltriméthylammonium, le chlorure de dodécyltriméthylammonium, le chlorure de méthyltributylammonium, le chlorure de méthyltributylammonium, l'hydrogénosulfate de méthyltributylammonium, le chlorure de méthyltricaprylylammonium, le chlorure de méthyltrioctylammonium, le bromure de myristyltriméthylammonium, le bromure de cétylpyridinium, le chlorure de phényltriméthylammonium, le borohydrure de tétrabutylammonium, le bromure de tétrabutylammonium, le chlorure de tétrabutylammonium, le fluorure de tétrabutylammonium, l'hydrogénosulfate de tétrabutylammonium, l'hydroxyde de tétrabutylammonium, l'iodure de tétrabutylammonium, le perchlorate de tétrabutylammonium, le bromure de tétraéthylammonium, le chlorure de tétraéthylammonium, l'hydroxyde de tétraéthylammonium, le bromure de tétrahexylammonium, l'iodure de tétrahexylammonium, le bromure de tétraméthylammonium, le chlorure de tétraméthylammonium, le fluorure de tétraméthylammonium, l'hydroxyde de tétraméthylammonium, l'iodure de tétraméthylammonium, le bromure de tétraoctylammonium, le bromure de tétrapropylammonium, le chlorure de tétrapropylammonium, l'hydroxyde de tétrapropylammonium, le chlorure de tétraéthylammonium, l'hydroxyde de tétraéthylammonium, le bromure de tétraméthylammonium, le chlorure de tétraméthylammonium, le fluorure de tétraméthylammonium, l'hydroxyde de tétraméthylammonium, le bromure de tétraoctylammonium, le bromure de tétrapropylammonium, le chlorure de tétrapropylammonium, l'hydroxyde de tétrapropylammonium, le chlorure de tributylméthylammonium et le chlorure de triéthylbenzylammonium.

8. Procédé selon l'une quelconque des revendications 5 et 6, dans lequel ledit catalyseur phosphonium quaternaire est sélectionné dans le groupe consistant en : le bromure de benzyltriphénylphosphonium, le chlorure de benzyltriphénylphosphonium, le bromure de butyltriphénylphosphonium, le chlorure de butyltriphénylphosphonium, l'acétate d'éthyltriphényl-phosphonium, le bromure d'éthyltriphénylphosphonium, l'iodure d'éthyltriphénylphosphonium, le bromure de méthyltriphénylphosphonium, le bromure de propyltriphénylphosphonium, le chlorure de propyltriphénylphosphonium, le bromure de tétrabutylphosphonium et le bromure de tétraphénylphosphonium.

9. Procédé selon l'une quelconque des revendications 5 et 6, dans lequel ledit éther-couronne est sélectionné dans le groupe consistant en le 18-couronne-6, le dibenzo-18-couronne-6, le dicyclohexyl-18-couronne-6, le 12-couronne-4, le 13-couronne-4, le 15-couronne-5 et le 27-couronne-9.

10. Procédé selon l'une quelconque des revendications 5 et 6, dans lequel ledit catalyseur polyéthylèneglycol est sélectionné dans le groupe consistant en l'éther diméthylique de monoéthylèneglycol, l'éther diméthylique de diéthylèneglycol, l'éther diméthylique de triéthylèneglycol, l'éther diméthylique de tétraéthylèneglycol, l'éther diméthylique de dipropylèneglycol, l'éther dibutylique de diéthylglycol, l'éther dibutylique de polyéthylèneglycol et l'éther dibutylique de diéthylèneglycol.

11. Procédé selon l'une quelconque des revendications 1-10, dans lequel ledit solvant est sélectionné dans le groupe consistant en un solvant organique et l'eau.

12. Procédé selon l'une quelconque des revendications 1-11, dans lequel ledit diol présente la stéréochimie suivante :

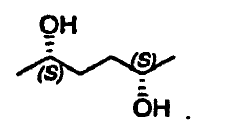

13. Procédé selon l'une quelconque des revendications 1-12, dans lequel ledit composé est le (*1S*,*4S*)-4-(4-hydroxy-1-méthyl-pentyloxy)-2-trifluorométhyl-benzonitrile ou un sel pharmaceutiquement acceptable correspondant.
